# EUROPEAN PATENT SPECIFICATION

(11) **EP 1 183 044 B1**
(45) Date of publication and mention of the grant of the patent: **10.10.2007**
(21) Application number: 00926717.0
(22) Date of filing: 12.05.2000
(51) Int. Cl.: A61K 39/39

(54) **ADJUVANT COMBINATIONS FOR IMMUNIZATION AND VACCINES**
ADJUVANSKOMBINATIONEN ZUR IMMUNISIERUNG UND VAKZINE
COMBINAISONS D'ADJUVANTS POUR L'IMMUNISATION ET LES VACCINS

(30) Priority: 12.05.1999 DK 65599
(43) Date of publication of application: 06.03.2002
(73) Proprietor: Statens Serum Institut, 2300 Copenhagen S (DK)
(72) Inventor: LINDBLAD, Erik, B., DK-1823 Frederiksberg (DK); ELHAY, Martin, J., Hawthorn, VIC 3122 (AU); ANDERSEN, Peter, DK-2700 Bronsh j (DK); BRANDT, Lise, Ostergaard, DK-1407 Copenhagen K (DK)
(74) Representative: Plougmann & Vingtoft A/S
(86) International application number: PCT/DK2000/000251
(87) International publication number: WO 2000/069458

(56) References cited:
- EP-A- 0 646 378
- WO-A-95/01441
- DE-C- 19 611 235
- US-A- 5 951 988
- DZATA G K ET AL: "Immunopotentiation of cattle vaccinated with a soluble Brucella abortus antigen with low LPS content: an analysis of cellular and humoral immune responses" VETERINARY MICROBIOLOGY, vol. 29, - 1991 pages 15-26, XP002901247
- BRANDT L ET AL: "ESAT-6 Subunit Vaccination against Mycobacterium tuberculosis" INFECTION AND IMMUNITY, vol. 68, no. 2, February 2000 (2000-02), pages 791-795, XP002901248

## Description

The present invention relates to adjuvant combinations comprising two or more different adjuvants. In particular the invention relates to adjuvant compositions comprising the adjuvants in aqueous media for immunization and vaccines.

The invention also relates to vaccines and immunization combination kits comprising two or more adjuvants and an antigenic substance.

### Background of the invention.

Since the English doctor Edward Jenner in 1796 discovered that the infectious agency causing cowpox in cattle was able to produce immunity against smallpox in human beings without causing serious illness, many efforts have been made in order to find other vaccines which can generate immunity against more or less severe diseases in animal and human beings without provoking the unpleasant, serious or fatal symptoms and reactions usually accompanying the ordinary diseases in question.

Thus, for example, tuberculosis in man has for many years been combated by vaccination with attenuated but living strains of Mycobacterium bovis (BCG vaccine). However, the efficacy of this procedure does not always provide satisfactory resistance to human tuberculosis in every population.

Therefore, attempts have been made instead to isolate and use fragments or subfragments of strains of human *Mycobacterium tuberculosis* as an immunogenic agent which when injected intradermally or subcutaneously in individuals would cause satisfactory Immunity against infections with naturally occurring strains of human *Mycobacterium tuberculosis.* On this basis, indeterminate substances from culture filtrates as well as a few isolated molecules such as Ag85 or ESAT-6 of *Mycobacterium tuberculosis* have been shown to provide some degree of immunity to tuberculosis. In the future it would be desirable to have vaccines based on well-defined substances which would always create high immunity against tuberculosis and other diseases.

Unfortunately, many highly purified substances, e.g. purified recombinant proteins, are not very immunogenic and do not generate an effective immune response protective against the real infectious disease. This fact has been recognised since the beginning of this century and it has been shown that low immunogenicity can be circumvented by combining the substance in question with immunogenic response potentiating agents, so-called adjuvants. A large number of such adjuvants and kind of adjuvants have been suggested but in general these have suffered from numerous problems that preclude their use in humans.

Document "Immunopotentiation of cattle vaccinated with a soluble Brucella abortus antigen with low LPS content: an analysis of cellular and humoral immune responses" by G. T. Dzata et al. in Veterinary Microbioly, vol. 29, 1991, pp.15-26, discloses an adjuvant combination comprising DDA-Br and trehalose dimycolate (TDM) or muramyl dipeptide (MDP) (hydrophobic second adjuvant components). The combination of DDA-Br and TDM with a soluble protein extract of gamma irradiated *Brucella abortus* strain 19 (SPEDA) induces higher serum antibody levels than SPEDA with DDA alone after vaccination of steers. Furthermore, the addition of TDM to DDA enhances the cell mediated immune responses, which is necessary to acquire resistance to brucellosis disease. The conclusion is that a combination of DDA and TDM potentiates CMI responses to a soluble B*.aborus* antigen preparation and may be useful as adjuvants for future vaccines.

### Disclosure of the Invention.

The present inventors have now discovered that two particular classes of adjuvants possess the capability to elicit a strong and long persisting immune response when administered in combination with an antigenic substance, even though this substance may have only poor immunogenicity per se.

Thus, the present invention relates to an adjuvant combination comprising a first adjuvant component which is a quaternary hydrocarbon ammonium halogenide of the formula NR¹R²R³R⁴-hal, wherein R¹ and R² independently each is a short chain alkyl group containing 1 to 3 carbon atoms, R³ and R⁴ independently each is a hydrocarbon group containing from 12 to 20 carbon atoms, preferably from 14 to 18 carbon atoms and hal is a halogen atom, and a hydrophobic second adjuvant component selected from the group consisting of: a monophosphoryl lipid, trehalose dibehenate, a sorbitan derivative and a triterpenoid saponin.

In the formula NR¹R²R³R⁴-hal the R¹ and R² groups may e.g. be methyl, ethyl, propyl and isopropyl, whereas R³ and R⁴ may be dodecyl, tridecyl, tetradecyl, pentadecyl, hexadecyl, heptadecyl, octadecyl nonadecyl and eicocyl groups. However, also other C₁₂-C₂₀ hydrocarbon groups are possible because even though the R³ and R⁴ groups usually and preferably are straight chain hydrocarbon groups they may in minor degree be branched having e.g. methyl and ethyl side chains. R³ and R⁴ may also have a minor degree of unsaturation, e.g. containing 1 - 3 double bonds each, but preferably they are saturated alkyl groups. R³ and R⁴ are preferably saturated alkyl groups containing from 14 to 18 carbon atoms.

The halogen atom "hal" is preferably bromine or chlorine because the other halogens, fluorine and iodine, may have undesirable biochemical, physiological and injurious effects, but for some experimental purposes, where such effects can be accepted, they may also be selected.

Especially preferred as the hydrophobic second adjuvant components are molecules binding to members of the so-called Toll-Like Receptor family (TLR), such as monophosphoryl lipids, lipoproteins or long chain bacterial polysaccharides. This is based on the hypothesis that signalling through receptors with limited variability present on cells of the innate immune system, triggered by molecules properly presented as part of an adjuvant complex, may elicit strong immune responses from the innate arm of the immune system which can serve to enhance subsequent cognate immunity. It has recently been shown that certain molecules of bacterial origin are recognised by cells of the innate immune system via a family of receptors known as the Toll-Like Receptors (TLR). This recent work and its implications are summarised in "Toll signaling pathways in the innate immune response." by K. V. Anderson in Current Opinion in Immunology, 2000 Feb;12(1):13-9 and "The Toll-receptor family and control of innate immunity." by E. B.Kopp and R. Medzhitov in Current Opinion in Immunology, 1999 Feb;11(1):13-8.

The monophosphoryl lipids are e.g. obtainable from microbial lipopolysaccharide (LPS) and are usually prepared from bacterial polysaccharides even though other microbial sources like viruses, moulds, fungi, yeasts and algae may be the source of origin for the phosphoryl lipid of choice. Suitable bacterial polysaccharides are e.g. described in "The Theory and Practical Applications of adjuvants" , chapter thirteen, pp. 287-313, Ed. by D.E.S. Stewart-Tull, 1995, John Wiley Sons Ltd., in "Methods in Microbiology" , Vol. 25, pp. 471-502, Ed. Stefan AE Kaufmann and Dieter Kabelitz, 1998, Academic Press, San Diego, California, USA and London, UK, and in "Vaccine" , vol. 15, No. 3, pp. 248-256, 1997, Elsevier Science Ltd., GB.

Also, the monophosphoryl lipids derivable from the microbial polysaccharides and suitable for use in the adjuvant combinations of the present invention are described in more details in the above references. The preferred monophosphoryl lipid is monophosphoryl lipid A (MPL-A) which is described in "The Theory and Practical Applications of adjuvants" , chapter thirteen, pp. 289-292, Ed. by D.E.S. Stewart-Tull, 1995, John Wiley Sons Ltd., in "Methods in Microbiology" , Vol. 25, pp. 483-484, Ed. Stefan AE Kaufmann and Dieter Kabelitz, 1998, Academic Press, San Diego, California, USA and London, UK, and in "Vaccine" , vol. 15, No. 3, p. 252, column 2, 1997, Elsevier Science Ltd., GB. The most preferred MPL-A is designated 3-O-deacylated monophosphoryl lipid A. However, also other derivatives of the MPL-A's may be applicable.

Examples of long chain bacterial polysaccharides, such as trehalose derivatives are described in "Methods in Microbiology", Vol. 25, p. 480, Ed. Stefan AE Kaufmann and Dieter Kabelitz, 1998, Academic Press, San Diego, California, USA and London, UK. A preferred long chain bacterial polysaccharide is trehalose dibehenate. Examples of muramyl dipeptide derivatives are described in "Methods in Microbiology"), Vol. 25, p. 484, Ed. Stefan AE Kaufmann and Dieter Kabelitz, 1998, Academic Press, San Diego, California, USA and London, UK. Especially preferred is N-acetylmuramyl-alanyl-isoglutamine.

Examples of block co-polymer adjuvants are described by e.g. Todd C.V. et al., Systematic development of a block copolymer adjuvant for trivalent influenza virus vaccine, Dev Biol Stand 1998; 92:341-51.

The adjuvant combination of the present invention may preferably be in the form of:
a) an aqueous composition comprising the quaternary hydrocarbon ammonium halogenide of the formula NR¹R²R³R⁴-hal, wherein R¹ and R² independently each is a short chain alkyl group containing 1 to 3 carbon atoms, R³ and R⁴ independently each is a medium chain length hydrocarbon group containing 12 to 20 carbon atoms and hal is a halogen atom, and
b) an aqueous composition comprising the hydrophobic second adjuvant component.

The aqueous media in these aqueous compositions may be any suitable aqueous solvent. However, formation of useful possible micelle structures appears to be sensitive to anions, like phosphate and sulphate ions. Thus, it is preferred that the adjuvant compositions of the inventions is formed in the absence or low levels of such ions.

The aqueous adjuvant compositions may be prepared by any suitable process or procedure, e.g. as described further on in the detailed part of this specification.

One preferred aspect of the aqueous adjuvant compositions of the present invention are compositions wherein the quaternary hydrocarbon ammonium halogenide is dimethyl dioctadecyl ammonium bromide or chloride (DDA-Br or DDA-Cl) and the second hydrophobic adjuvant component is a monophosphoryl lipid, most preferably MPL-A.

Another preferred aspect of the aqueous adjuvant compostions of the present invention are compositions wherein the quaternary hydrocarbon ammonium halogenide is dimethyl dioctadecyl ammonium bromide or chloride (DDA-Br or DDA-Cl) and the hydrophobic second adjuvant component is a long chain bacterial polysaccaride, such as a trehalose derivative, most preferably trehalose dibehenate.

Also preferred as an aqueous adjuvant composition of the present invention is a composition wherein the quaternary hydrocarbon ammonium halogenide is dimethyl dioctadecyl ammonium bromide or chloride (DDA-Br or DDA-Cl) and the hydrophobic second adjuvant component is a triterpenoid saponin.

Furthermore aqueous adjuvant compositions wherein the quaternary hydrocarbon ammonium halogenide is dimethyl dioctadecyl ammonium bromide or chloride (DDA-Br or DDA-Cl) and the hydrophobic second adjuvant component is a sorbitan derivative, preferably sorbitan trioleate, are preferred.

If expedient, the different adjuvant compositions may be combined into one single composition either as a stock composition or Immediately before use.

The invention concerns also a kit for immunization, said kit comprising a first adjuvant component which is a quaternary hydrocarbon ammonium halogenide of the formula NR¹R²R³R⁴-hal, wherein R¹ and R² independently each is a short chain alkyl group containing 1 to 3 carbon atoms, R³ and R⁴ independently each is a hydrocarbon group containing from 12 to 20 carbon atoms, preferably from 14 to 18 carbon atoms, and hal is a halogen atom, and a hydrophobic second adjuvant component, and an antigenic selected from the group consisting of : a monophosphoryl lipid, trehalose dibehenate, a sorbitan derivative and a triterpenoid saponin. substance.

Preferred kits are kits prepared from the above described preferred aqueous adjuvant compositions and an antigenic substance.

Such kit may be presented in the form of individual containers or compartments containing the different adjuvants and the antigenic substance and any solvent necessary for effecting the immunization procedure as well as any necessary device for the performance thereof. If appropriate the adjuvants and the antigenic substance may also be combined and stocked in one single container. If the adjuvants and the antigenic substance each is contained in a separate container they may be mixed in any order before use. For some applications it may be advantageous, however, to mix the adjuvants and the antigenic substances in a particular order for obtaining optimum results.

In principle the antigenic substance may be any pure chemical species such as a protein or a fragment thereof or artificial mixtures prepared of such species. But it can also be any naturally occuring mixture of chemical species such as e.g. a cell homogenate or fractions thereof, a culture filtrate from microorganisms or cell tissues from multicellular organisms, e.g. higher animals.

Specifically the antigenic substance may be derived from a culture of metabolising *Mycobacterium tuberculosis, Mycobacterium bovis* and other environmental *Mycobacteria* such as e.g. *Mycobacterium avium.* A particular interesting substance from the filtrate of such Mycobacteria is the ESAT-6 protein (Early Secretory Antigenic Target) which is a dominant target for cell mediated immunity in the early phase of tuberculosis in TB patients and in different animal models. It contains 95 amino acid residues and has a deduced molecular mass of approximately 10 kDa. Its immunogenecity per se is low, but in combination with the adjuvant combinations of the present invention it has turned out to be a potent candidate for provoking high and persisting immunity against tuberculosis as is demonstrated in the following detailed part of this specification. Another interesting antigenic substance is a hybrid molecule comprising ESAT-6 from *Mycobacterium tuberculosis.*

ESAT-6 as well as many other antigens applicable in combination with the adjuvant combinations of the present invention, today can be produced artificially, e.g. synthetically or by genetic recombinant techniques.

In addition to provide immunity to diseases the adjuvant combinations of the present invention can also be used for producing antibodies against compounds which are poor immunogenic substances per se and such antibodies can be used for the detection and quantification of the compounds in question, e.g. in medicine and analytical chemistry.

Without being bound by theory it is believed that an adjuvant such as DDA, which induces strong CMI (cell mediated Immune) responses, has the ability to form micelles in aqueous solutions. This structure provides a matrix for the retention of other hydrophobic immunomodulatory compounds and the formation of composite micelles with both increased adjuvant activity and decreased diffusability.

Preferred embodiments of the adjuvant combination and the immunization combination kit of the present invention are set forth in the dependent claims in the accompanying set of claims attached.

The invention will now be further described and illustrated by reference to embodiment examples of the invention and experimental examples forming the basis for the discovery of the present invention.

### Materials and Methods

### Animals

The studies were performed with 8 to 12 weeks old C57BU6 (C57BL/6J, H-2^{b}) female mice, purchased from Bomholtegaard, Ry, Denmark.

Infected animals were housed in cages contained within a laminar flow safety enclosure.

### Bacteria

*M. tuberculosis* H37Rv was grown at 37°C on Löwenstein Jensen medium or in suspension in modified Sauton medium enriched with 0.5% sodium pyruvate and 0.5% glucose.

### Adjuvants

**DDA-Br** (In the following abbreviated to DDA; Eastman Kodak, Inc., Rochester, N.Y.) is mixed into sterile water to a concentration of 2.5 mg/ml and heated to 80 °C while stirring continuously on a magnetic stirring hotplate for 10 min and then cooled to room temperature before use.

**MPL-A** (In the following abbreviated to MPL) (MPL, Ribi Immunochem, Hamilton, MT, USA) is mixed into sterile water containing 2 µl triethylamine to a concentration of 1 mg/ml. The mixture is heated in a 65-70 °C water bath for 30 seconds and then sonicated for 30 seconds. The heating and sonicating procedure is repeated twice. The solution is stored at 4 °C until use.

**D-(+)-Trehalose 6, 6' dibehenate** (In the following abbreviated to TDB, Sigma Biochemicals, St. Louis, MO, USA) is mixed into sterile water containing 2 % dimethyl sulfoxide to a concentration of 0.5 mg/ml. The mixture is then sonicated for 30 seconds. The sonicating procedure is repeated twice. The solution is stored at -20 °C until use.

N-acetylmuramyl-L-alanyl-D-isoglutamine (In the following abbreviated to AMI, Sigma Biochemicals, St. Louis, MO, USA) is mixed into sterile water to a concentration of 1 mg/ml. The mixture is then sonicated for 30 seconds. The sonicating procedure is repeated twice. The solution is stored at 4 °C until use.

**Saponin** (Sigma Biochemicals, St. Louis, MO, USA) is mixed into sterile water to a concentration of 1 mg/ml. The mixture is then mixed by vortexing for 30 seconds. The vortexing procedure is repeated twice. The solution is stored at 4 °C until use.

**Sorbitan trioleate** (In the following abbreviated to sorbitol, Sigma Biochemicals, St. Louis, MO, USA) is mixed into sterile water to a concentration of 10% weight per volume. The mixture is then mixed by vortexing for 30 seconds. The vortexing procedure is repeated twice. The solution is stored at 4 °C until use.

On the moming of immunization the antigen is mixed with saline and the second adjuvant component is added. Then DDA is added and the suspension is mixed on a vortex mixer.

### Immunization

Mice were immunized subcutaneously (sc) two or three times at two weekly intervals, In the back with the experimental vaccines which contained either 50 µg ST-CF/dose, 10-50 µg ESAT- 6/dose or 1 µg /dose of an ESAT-6 containing subunit vaccine, emulsified in DDA (250µg/dose, Eastman Kodak, Inc., Rochester, N.Y.) with or without 25 µg monophosphoryl lipid A (MPL, Ribi Immunochem, Hamilton, MT,USA), 50 micrograms D-(+)-Trehalose 6,6' dibehenate (T2268, Sigma Biochemicals, St. Louis, MO, USA), 25 micrograms N-acetylmuramyl-L-alanyl-D-isoglutamine (A9519, Sigma Biochemicals, St. Louis, MO, USA), 2.5 micrograms Saponin (S7900, Sigma Biochemicals, St. Louis, MO, USA) or 1 % weight per volume Sorbitan trioleate (S7135, Sigma Biochemicals, St. Louis, MO, USA), in a volume of 0.2 ml.

A single dose of BCG Copenhagen 1331 (5x10⁴ cfu (colony forming units)) was injected subcutaneously at the base of the tail in control animals.

### Experimental infections

Intravenous (iv) infections were administered via the lateral tail vein with an inoculum of 5 x 10⁴ *M. tuberculosis* (H37Rv) suspended in phosphate buffered saline (PBS) in a volume of 0.1 ml. The mice were sacrificed two weeks later.

Respiratory infections (ri) of the animals with *M.tuberculosis* (Erdman) were administered by the aerosol route with an inoculum of 5x10⁶/ml. Six weeks later the mice were sacrificed. Bacterial numbers in the liver, spleen or lung were determined by double serial 3 fold dilutions of individual whole organ homogenates on Middlebrook 7H11 medium. Organs from the BCG vaccinated animals were grown on medium supplemented with 2µg 2-thiophene-carboxylic acid hydrazide (TCH). Colonies were counted after 3 weeks of incubation at 37°C. The protective efficacies are expressed as means of the bacterial counts in immunized mice after subtraction of the adjuvant control obtained from 5 animals/group.

### Mycobacterial antigens

Short-term culture filtrate (ST-CF) was produced as described previously {Andersen P., Askgaard D., Ljungqvist L., Bennedsen J., and Heron I., Proteins released from Mycobacterium tuberculosis during growth. Infect. Immun. 59: 1905-1910, 1991}. Briefly, *M tuberculosis* (8 x 10⁶ CFU/ml) were grown in modified Sauton medium without Tween 80 on an orbital shaker for 7 days. The culture supernatants were sterile filtered and concentrated on an Amicon YM3 membrane (Amicon, Danvers, MA.)

Recombinant ESAT-6 was prepared by Brandt et al. {Brandt L., Oettinger T., and Andersen P., Key epitopes on the ESAT-6 antigen recognized in mice during the recall of protective immunity to Mycobacterium tuberculosis. J. Immunol. 157:3527-3533, 1996}

A recombinant subunit molecule containing ESAT-6 and Ag85B (hereafter referred to Hybrid) was prepared as described {Olsen, A., van Pinxteren. L., Arend, S., Okkels, L., Rasmussen, P., Ottenhoff, T. and Andersen, P. An efficient tuberculosis subunit vaccine based on a fusion protein of Ag85B and ESAT-6. J. Immunol., submitted}

The LPS content in the preparations was measured by the LAL test to be below 0.3 ng/µg protein and this concentration had no influence on cellular activity. The protein was kept at - 80°C until use.

### Lymphocyte cultures

Lymphocytes from spleens were obtained as described previously {Andersen P., Askgaard D., Ljungqvist L., Bentzon M.W., and Heron I., T-cell proliferative response to antigens secreted by Mycobacterium tuberculosis. Infect. Immun. 59: 1558-1563, 1991}. Blood lymphocytes were purified on density medium. Cells pooled from 3-5 mice in each experiment were cultured in microtiter wells (96 well, Nunc, Roskilde, Denmark) containing 2 x 10⁵ cells in a volume of 200 µl RPMI 1640 supplemented with 2-mercaptoethanol, Penicillin-Streptomycin, glutamine and 5% (vol/vol) FCS (foetal calf serum). ST-CF and the preparations of ESAT-6 were used in various concentrations (0.5-20 micrograms/ml) in the cultures. Culture filtrate fractions were used at 5 micrograms/ml. Based on previous dose-response investigations, purified mycobacterial antigens and the peptides were all used at 0.5-10 micrograms/ml. Con A at a concentration of 1 microgram/ml was used in all experiments as a positive control for cell viability. All preparations were tested in cell cultures and found to be non-toxic at the concentrations used in the present study. Supernatants were harvested from parallel cultures for the investigation of cytokines after 72 h of incubation.

### IFN-γ EUSA

Microtiter plates (96 well, maxisorb, Nunc) were coated with monoclonal Hamster anti-murine IFN-γ (Genzyme, Cambridge, Ma) in PBS at 4°C. Free bindings site were blocked with 1% (wt/vol) BSA / 0.05% Tween 20. Culture supernatants were tested in triplicates and IFN-γ was detected by biotin-labeled rat anti-murine monoclonal antibody (clone XMG1.2, Pharmingen, San Diego, Ca.). Recombinant IFN-γ (Pharmingen) was used as a standard.

### EUSPOT technique

In this assay microtiter plates (96 well, maxisorb) were coated with 2.5 micrograms/ml of monoclonal hamster anti-murine IFN-γ (Genzyme). Free binding sites were blocked with bovine serum albumin followed by washing with PBS/0.05% Tween 20. Analyses were always conducted on cells pooled from three mice. Cells were stimulated with optimal concentrations of antigen in modified RPMI 1640 for 18-22 h and subsequently cultured without antigen for 7 h directly in the ELISPOT plates. The cells were removed by washing and the site of cytokine secretion detected by biotin-labeled rat anti-murine IFN-γ monoclonal antibody (clone XMG1.2, Pharmingen) and phosphatase-conjugated streptavidin (Jackson ImmunoResearch Lab., Inc., PA.). The enzyme reaction was developed with BCIP (Sigma). Blue spots were counted microscopically. The relationship between the number of cells per well and the number of spot was linear in concentrations, 2x10⁵ - 6.2x10³ cells/well. Wells with less than 10 spots were not used for calculations.

### ESAT-6 specific IgG ELISA

ELISA plates (NUNC Maxisorp, type 96F) were coated with ESAT-6 (0.1 micrograms/ well) overnight at 4° C. Free binding sites were blocked with 1 % bovine serum albumin-PBS. Individual mouse sera from 5 mice/group were analyzed in three-fold dilutions. IgG (P260, diluted 1/1000, DAKO) antibody was detected by peroxidase-conjugated rabbit anti-mouse reagent. Antibody titers are expressed as reciprocal end-point titer.

### Statistical methods

Mean response to individual antigens were compared by the paired Student's *t*ttest.
The efficacies of different vaccination protocols have been compared by one-way analysis of variance of log 10 cfu (colony forming units).

### Example 1

### ESAT-6 is highly recognized during infection but not after subunit vaccination, i.e. ESAT-6 is a low immunogenic molecule

To study the immune response after vaccination with ESAT-6, C57BL/6 mice were vaccinated with an ESAT-6 subunit vaccine emulsified in DDA; an adjuvant which has been shown to induce an immune response of the Th1 type {Grun. J.L. and Maurer, P. H. Different T helper cell subsets elicited in mice utilizing two different adjuvant vehicles, Cell Immunol., 121: 134-145, 1989*;* Lindblad, E.B., Elhay, M. J. Silva, R., Appelberg, R and Andersen, P., Adjuvant modulation of immune response to tuberculosis sub-unit vaccines. infect. immun., 65(2): 623-629, 1997}. in parallel, groups of mice were immunized with experimental vaccines consisting of Short Term Culture Filtrate (ST-CF). All vaccines were given three times with a two week interval. One group of BCG vaccinated mice and one group of mice given a primary infection with *M.tuberculosis* were also included. The IFN-γ contents in 72 h supernatants after stimulation of splenocytes from TB infected mice with ST-CF or ESAT-6 *in vitro* are shown in Table 1, column 2. Stimulation with ST-CF exhibited a powerful IFN-γ response of 34,000 pg/ml followed up by a pronounced response to ESAT-6 (~19700 pg/ml).

The ability of the subunit vaccinations to generate an antigen specific immune response to the homologue preparation were investigated three weeks after the last booster injection by stimulating cells from the draining lymph nodes *in vitro,* Table 1, column 3. Of the immunized mice, the group of ST-CF vaccinated induced the strongest IFN-γ release after stimulation with the homologue preparation (~19,150 pg/ml). In contrast, no ESAT-6 specific IFN-γ response was detectable (< 50 pg/ml).

**Table 1**

| | | |
|---|---|---|
| | ^{a}IFN-γ recall responses | |
| ^{b}Antigen | ^{c}TB infection | ^{d}Vaccination |
| Unstimulated | 1.405 ± 25 | 125 ± 25 |
| ST-CF | 34,317 ± 972 | 19,156 ± 987 |
| ESAT-6 | 19,668 ± 3281 | <50 |

| | | |
|---|---|---|
| ^{a}Recall responses are expressed as mean IFN-γ contents representing the mean of triplicate values in pg/ml ± SEM in the supernatant monitored after 72 h. of *in vitro* stimulation. ^{b}Mice were vaccinated with BCG, ST-CF (50 µg/dose), or ESAT-6 (20 µg/dose) using DDA as adjuvant. ^{c}Spleen cells from mice infected i.v. with 5x10⁴ cfu *M.tuberculosis* were isolated two weeks postinfection and a pool of cells from 5 mice was stimulated *in vitro* for 72 h whereafter the IFN-γ (pg/ml) contents in the supernatants were determined. ^{d}Lymph node cells were isolated three weeks after the last booster injection and a pool of cells from 5 mice was stimulated *in vitro* with the homolog protein. IFN-γ responses < 50 were not detectable in this assay. | | |

### No protection obtained after Immunization with ESAT-6 in DDA

To study the protective efficacy of these experimental vaccines mice were left for three months after which the mice received either iv. or aerosol infection with live *M.tuberculosis.* The bacterial load in liver or lung were determined and the protective efficacy of these vaccines are expressed as the log₁₀ reduction compared to the control mice, shown for two individual experiments in Table 2 (Expt. I and Expt. II). Vaccinations with either BCG or ST-CF/DDA evoked a significant protection of 0.73 log₁₀ and 1.10 log₁₀, respectively, compared to control mice, whereas no significant reduction of the bacterial load was detected after ESAT-6 vaccination, shown in Table 2, Expt. I. In experiment II the protective efficacy of BCG and ST-CF vaccination were confirmed. The ESAT-6/DDA vaccine induced only minimal levels of protection.

**Table 2**

| | ^{a}Protection | |
|---|---|---|
| ^{b}Vaccination | ^{c}Expt. I | ^{d}Expt. II |
| BCG | 0.73 ± 0.06 (*p*= 0.008) | 0.98 ± 0.22 (*p*= 0.008) |
| ST-CF | 1.10 ± 0.09 (*p*= 0.008) | 0.81 ± 0.17 (*p*= 0.006) |
| ESAT-6 | 0.11 ± 0.10 | 0.25 ± 0.09 (*p*= 0.009) |

| | | |
|---|---|---|
| ^{a}The protective efficacies of vaccines are expressed as the log₁₀ reduction of the bacterial load. Data expressed are means based on duplicate analysis for each group (n=5). *P* values have been given for bacterial load that are significantly different from numbers found for unimmunized control mice determined by Student's *t*-test. ^{b}Mice were BCG vaccinated or immunized s.c. with experimental vaccines emulsified in DDA. ^{c}Protection obtained in the liver of C57BU6 mice after TB infection given iv. ^{d}Protection obtained in the lung after aerosol TB infection. | | |

### Example 2

### DDA combined with MPL promotes an efficient response to ESAT-6

It is a general accepted fact that adjuvants have some selectivity for the induction of a certain dass of immune response. Since the importance of a Th1 cytokine release based on IFN-γ production has been shown to be essential in the resistance to TB {Flynn J.L., Chan J., Triebold K.J., Dalton D.K., Stewart T.A., and Bloom B.R., An essential role for interferon gamma in resistance to Mycobacterium tuberculosis infection, j.Exp.Med.,178:2249-2254,1993; and Cooper A.M., Dalton D.K., Stewart T.A., Griffen J.P., Russel D.G., and Orme I., M. Disseminated tuberculosis in interferon gamma gene-disrupted mice, J.Exp.Med., 178:2243-2247,1993}, MPL was added to the ESAT-6/DDA preparation In the attempt to investigate the potential of this vaccine. Mice were Immunized three times and one week after the 3^{rd} vaccination the ESAT-6 specific immune responses of blood cells were investigated. Only minimal T cell recognition of ESAT-6 could be detected from mice which received ESAT-6 mixed with DDA. In contrast, immunization with ESAT-6 emulsified in DDA+MPL (i.e. DDA-Br+MPL-A) generated a very potent IFN-γ response and very high frequencis of cells secreting IFN-γ (1: 470), measured by the sensitive ELISPOT technique, Table 3.

The development of the ESAT-6 specific antibody response was investigated in mice 7 weeks after the first vaccination shown in Table 3. High titers of ESAT-6 specific lgG are present in the sera from mice vaccinated with ESAT-6/DDA+MPL compared with the amounts found in sera after ESAT-6/DDA vaccination.

**Table 3**

| | ESAT-6 specific recall response | | | | |
|---|---|---|---|---|---|
| | Expt.I | | | Expt. II | |
| ^{a}Vaccine | ^{b}IFN-γ | ^{c}Frequency | ^{d}ESAT-6 specific IgG | ^{c}Frequency | ^{d}ESAT-6 specific IgG |
| DDA | <50 | < 1:20,000 | <3 | < 1:20,000 | < 3 |
| DDA+MPL | <50 | < 1:20,000 | < 3 | < 1:20,000 | < 3 |
| | | | | | |
| ESAT-6 + DDA | < 50 | 1: 14,800 | 32,768 | 1: 8,700 | 65,530 |
| ESAT-6 + MPL | < 0.05 | 1:14800 | 32768 | 1:10,000 | ND |
| ESAT-6 + DDA/MPL | 12,235 ± 553 | 1: 470 | 262,144 | 1: 440 | 524,288 |

| | | | | | |
|---|---|---|---|---|---|
| ^{a} Mice were vaccinated with adjuvant or ESAT-6 subunit vaccines (10 µg /dose) three times with a 2 week interval. ^{b} Means of the IFN-γ responses in pg/ml measured In 72 h. supernatant of blood cell cultures one week after the last booster injection ^{c} Frequencies of IFN-γ producing lymphocytes were estimated by ELISPOT analysis of blood cells established from immunized mice 1 week after the last booster injection. Frequencies were estimated from a pool of 5 mice. The results are expressed as mean of duplicate values and the difference between duplicate cultures are <12 % of the mean. Frequencies lower than 1:20,000 were not detectable in this assay. ^{d} 5 weeks after the primary vaccination, sera were determined by ELISA as described in Materials and Methods. The data listed are the end-point titers of a pool of sera from 5. The results listed are mean of dublicate values and the difference between dublicate wells are < 11 %. | | | | | |

### Example 3

### Protective efficacy of the ESAT-6 vaccine.

In order to measure the efficacies of the ESAT-6/DDA+MPL vaccine the mice were challenged with an aerosol administration of *M.tuberculosis* (Erdman) 6-8 weeks after the last ESAT-6 immunization and 6 weeks post infection the spleen and lung were harvested for determination of the bacterial load. As shown in Table 4 the protection obtained from ESAT-6 /DDA+MPL vaccination was similar to that induced by BCG vaccination with no significant difference in any of the two experiments described. In contrast, the ESAT-6/DDA vaccine did not protect significantly compared to the unvaccinated control, either in the spleen or in the lung.

**Table 4**

| Experiment | ^{a}Vaccine | ^{b}Log₁₀ resistance | |
|---|---|---|---|
| | | Spleen | Lung |
| I | | | |
| | DDA | <0.05 | < 0.05 |
| | MPL | <0.05 | < 0.05 |
| | DDA+MPL | <0.05 | < 0.05 |
| | ESAT-6 / DDA | ^{c}0.28 (± 0.14) | 0.18 (± 0.12) |
| | ESAT-6 / DDA+MPL | ^{c}0.58 (± 0.10) | ^{c} 0.41 (± 0.05) |
| | BCG | ^{c} 0.53 (± 0.19) | ^{c} 0.77 (± 0.06) |
| | | | |
| II | DDA+MPL | < 0.05 | < 0.05 |
| | ESAT-6/ DDA+MPL | ^{c}0.89 (± 0.32) | ^{c} 0.50 (± 0.09) |
| | BCG | ^{c} 0.94 (± 0.24) | ^{c} 0.46 (± 0.09) |

| | | | |
|---|---|---|---|
| ^{a} Mice were vaccinated s.c.with ESAT-6 subunit vaccines (10 µg /dose) three times with 2 weeks intervals or BCG vaccinated (4x10⁵ cfu). ^{b} The bacterial load is expressed as the log₁₀ reduction measured in the spleen and lung 6 weeks after aerosol challenge. ^{c}Protective efficacies which are significantly different from control mice, determined by Student's *t*-test. | | | |

### Example 4

### Enhancement of the protective efficacy of adding MPL to vaccines based on highly Immunogenic molecules.

Vaccination of C57BL mice with ST-CF emulsified in DDA has been shown to induce a protection at levels comparable to BCG {Andersen P., Effective vaccination of mice of mice against Mycobacterium tuberculosis infection with a soluble mixture of secreted mycobacterial proteins., infect immun. 62: 2536-2544, 1994}.

To analyse the protective capacity of vaccines consisting of ST-CF in DDA+MPL we immunized C57BL mice with ST-CF mixed in this vaccine. None of the adjuvants alone raised a protective immune response (data not shown), neither did ST-CF emulsified in MPL alone, Table 5. After adding MPL to the ST-CF/DDA vaccine the IFN-γ response of this highly immunogenic antigen preparation was enhanced a 10 fold, and the protective efficacy was also significantly enhanced compared to a similar vaccine mixed with DDA alone. This demonstrated a lack of efficacy using MPL on its own but at the same time showed that it can be an effective coadjuvant to DDA, promoting an efficient protective immune response.

**Table 5**

| ^{a}Vaccine | ^{b}IFN-γ (pg/ml) | ^{c}Log₁₀ resistance |
|---|---|---|
| MPL+ ST-CF | 1003 | 0.10 ± 0.08 |
| DDA+ST-CF | 458 | 0.69 ± 0.10 |
| MPL+DDA+ST-CF | 4936 | 0.93 ± 0.11 |
| BCG | 588 | 1.29 ± 0.09 |

| | | |
|---|---|---|
| ^{a}Mice were vaccinated s.c. with experimental subunit vaccines (100 µg /dose) twice with 2 weeks intervals or BCG vaccinated (4x10⁵ cfu). ^{b} Splenocytes were stimulated in vitro with ST-CF (4µg/ml) and the IFN-γ contents were measured In 72h. supernatants. The results are expressed as mean of duplicate values and the difference between duplicate cultures are < 6 % of the mean. IFN-γ responses < 50 were not detectable in this assay. ^{c}The bacterial load after i.v. challenge expressed as the log₁₀ reduction measured In the spleen compared to unvaccinated control. | | |

### Example 5

### Demonstration of the principle of enhanced immunogenicity of combined adujuvant compositions using a selection of other secondary adjuvants.

To demonstrate that the principle of enhanced adjuvanticity is not simply restricted to the combination of DDA plus MPL, several other molecules meeting the criteria laid out above for effective co-adjuvanticity of second adjuvants were tested. The molecules chosen were:
D-(+)-Trehalose 6, 6' dibehenate, Sigma Biochemicals, T2268 (TDB) N-acetylmuramyl-L-alanyl-D-isoglutamine, Sigma Biochemicals, A9519 (AMI) Saponin, Sigma Biochemicals, S7900 (Saponin) Sorbitan trioleate, Sigma Biochemicals, S7135 (Sorbitol)

Since the magnitude of the immune responsive to ESAT-6 was shown to be highly dependant on the composition of the adjuvant (see above), C57BU6 mice were vaccinated with an ESAT-6-containing subunit vaccine emulsified in DDA plus the coadjuvant molecules described immediately above. In parallel, mice were immunized with the same antigen in a DDA/MPL combination prepared as previously described. All groups received two vaccinations, two weeks apart. Control animals were infected with a single dose of BCG Copenhagen 1331 (5x10⁴ cfu (colony forming units)) injected subcutaneously at the base of the tail.

The IFN-γ levels in 72 h supernatants after stimulation of splenocytes from vaccinated mice *In vitro* are shown in Table 6. Stimulation with specific antigen induced a strong IFN-γ response in the test vaccinated groups comparable to that obtained with the DDA/MPL. This demonstrates that the principles laid out in the patent application can be used to produce enhanced immune responses using a variety of second adjuvant components, and are efficacious at stimulating responses to a range of defined or complex antigens

**Table 6**

| ^{a}Vaccine | ^{b}IFN-γ (pg/ml) |
|---|---|
| Experiment 1 | |
| | |
| Control | 189 |
| DDA + ^{c}Hybrid | 1,440 |
| DDA + MPL+ Hybrid | 11,324 |
| | |
| Experiment 2 | |
| Control | 660 |
| DDA + MPL+ Hybrid | 14,006 |
| DDA + TDB+ Hybrid | 15,768 |
| DDA + AMI+ Hybrid | 14,514 |
| DDA + Saponin+ Hybrid | 14,314 |
| DDA + Sorbitol+ Hybrid | 6,635 |
| BCG | 720 |

| | |
|---|---|
| ^{a}Mice were vaccinated s.c. with experimental subunit vaccine (1 µg /dose) twice with a 2 week interval or BCG vaccinated (4x10⁵ cfu). ^{b} Splenocytes were stimulated in vitro with experimental subunit vaccine (1.25-10 µg/ml) and the IFN-γ contents were measured in 72h. supernatants. The results are expressed as mean of duplicate values and the difference between duplicate cultures are < 10 % of the mean. IFN-γ responses < 50 were not detectable in this assay. ^{c}The experimental subunit vaccine used contained both ESAT-6 and Ag85B and is referred to as Hybrid. | |

### Concluding remarks

The selection of adjuvants having no or low levels of undesired side effects are quite limited. Al(OH)₃, which is known to prime mainly a Th2 type immune response, is the only adjuvant which is licensed for human use today. All of the adjuvant components tested are mild adjuvants and may therefore be potential candidates for human use. As shown in the above studies, antigen mixed with a combination of DDA plus an appropriate immunomodulator clearly demonstrates that this combination amplifies some of the crucial events in the protective immunological cascade after vaccination. These findings imply that a vaccine based even on molecules of low immunogenicity (such as ESAT-6) used in combination with DDA + and an appropriate second adjuvant can be a highly efficient way of triggering the right type of immune response.

The use of this new combination of adjuvants has been demonstrated using TB antigens, but the use of this combination may be of great value for other antigens found outside the TB field.

## Claims

1. An adjuvant combination which comprises:
- a first adjuvant component which is a quaternary hydrocarbon ammonium halogenide of the formula NR¹R²R³R⁴-hal, wherein R¹ and R² Independently each is a short chain alkyl group containing 1 to 3 carbon atoms, R³ and R⁴ Independently each is a hydrocarbon group containing from 12 to 20 carbon atoms, preferable from 14 to 18 carbon atoms, and hal is a halogen atom, and
- a hydrophobic second adjuvant component selected from the group consisting of: a monophosphoryl lipid, trehalose dibehenate, a sorbitan derivative and a triterpenold saponin.

2. An adjuvant combination according to claim 1, which comprises:
- a first adjuvant component which is the quaternary hydrocarbon ammonium halogenide dimethyl dioctadecyl ammonium bromide or chloride (DDA-Br or DDA-CI), and
- a hydrophobic second adjuvant component selected from the group consisting of: monophosphoryl lipid A (MPL-A), trehalose dibehenate (TDB) and a sorbitan derivative.

3. The adjuvant combination of claim 1 or 2, wherein the sorbitan derivative is sorbitan trioleate.

4. An immunization combination kit comprising an adjuvant combination according to any of claims 1-3 and an antigenic substance.

5. The immunization kit according to claim 4, wherein the antigenic substance is derived from a cell homogenate or fractions thereof.

6. The immunization kit according to claim 4, wherein the antigenic substance is a culture filtrate (CF), such as a culture filtrate stemming from the cultivation of a micro-organism.

7. The Immunization kit according to claim 4, wherein the antigenic substance is derived from *Mycobacterium tuberculosis.*

8. The Immunization kit according to claim 4, wherein the antigenic substance is an antigenic fragment, e.g. a substantially pure molecular species.

9. The Immunization kit according to claim 4, wherein the antigenic molecular species is of synthetic or recombinant origin.

10. The Immunization kit according to claim 4, wherein the antigenic molecular species is ESAT-6 from *Mycobacterium tuberculosis.*

11. The immunization kit of according to claim 4, wherein the antigenic molecular species is a recombinant hybrid molecule comprising ESAT-6 from *Mycobacterium tuberculosis.*

## Patentansprüche

1. Adjuvanskombination, umfassend:
- eine erste Adjuvanskomponente, die ein quaternäres Kohlenwasserstoffammoniumhalogenid mit der Formel NR¹R²R³R⁴-hal ist, wo jedes R¹ und R² unabhängig eine 1 bis 3 Kohlenstoffatome enthaltende kurzkettige Alkylgruppe ist, jedes R³ und R⁴ unabhängig eine von 12 bis 20 Kohlenstoffatomen, vorzugsweise von 14 bis 18 Kohlenstoffatomen, enthaltende, Kohlenwasserstoffgruppe ist, und hal ein Halogenatom ist, und
- eine hydrophobische zweite Adjuvanskomponente, die aus der Gruppe bestehend aus: einem Monophosphoryllipid, Trehalose Dibehenat, einem Sorbitanderivat und einem Triterpenoid-Saponin ausgewählt ist.

2. Adjuvanskombination nach Anspruch 1, umfassend:
- eine erste Adjuvanskomponente, die das quaternäre Kohlenwasserstoffammoniumhalogenid Dimethyldioctadecylammoniumbromid oder -chlorid (DDA-Br oder DDA-Cl) ist, und
- eine hydrophobische zweite Adjuvanskomponente, die aus der Gruppe bestehend aus: Monophosphoryllipid A (MPL-A), Trehalose Dibehenat (TDB) und einem Sorbitanderivat ausgewählt ist.

3. Adjuvanskombination nach Anspruch 1 oder 2, wo das Sorbitanderivat Sorbitantrioleat ist.

4. Immunisierungskombinationskit umfassend eine Adjuvanskombination nach irgendeinem der Ansprüche 1-3 und eine antigene Substanz.

5. Immunisierungskit nach Anspruch 4, wo die antigene Substanz aus einem Zellhomogenat oder Fraktionen davon abgeleitet ist.

6. Immunisierungskit nach Anspruch 4, wo die antigene Substanz ein Kulturfiltrat (CF) ist, wie z.B. ein Kulturfiltrat stammend aus der Kultivierung eines Mikroorganismus.

7. Immunisierungskit nach Anspruch 4, wo die antigene Substanz von *Mycobacterium tuberculosis* abgeleitet ist.

8. Immunisierungskit nach Anspruch 4, wo die antigene Substanz ein antigenes Fragment ist, z.B. eine im wesentlichen reine molekulare Spezies.

9. Immunisierungskit nach Anspruch 4, wo die antigene molekulare Spezies synthetischen oder rekombinanten Ursprungs ist.

10. Immunisierungskit nach Anspruch 4, wo die antigene molekulare Spezies ESAT-6 von *Mycobacterium tuberculosis* ist.

11. Immunisierungskit nach Anspruch 4, wo die antigene molekulare Spezies ein rekombinantes Hybridmolekül ist, umfassend ESAT-6 von *Mycobacterium tuberculosis.*

## Revendications

1. Combinaison adjuvant, comprenant:
- un premier composant d'adjuvant qui est un halogénure d'ammonium hydrocarbure quaternaire de la formule NR¹R²R³R⁴-hal, dans laquelle R¹ et R² sont indépendamment un groupe alkyle à chaine courte contenant de 1 à 3 atomes de carbone, R³ et R⁴ sont indépendamment un groupe hydrocarbure contenant de 12 à 20 atomes de carbone, de préférence de 14 à 18 atomes de carbone, et hal est un atome d'halogène, et
- un second composant adjuvant hydrophobique, choisi dans le groupe constitué: d'un lipide monophosphoryle, du dibéhénate de tréhalose , d'un dérivé de sorbitan et d'un triterpénoïde saponine.

2. Combinaison adjuvant selon la revendication 1, comprenant:
- un premier composant adjuvant qui est l'halogénure d'ammonium hydrocarbure quaternaire, bromure ou chlorure de diméthyl-dioctadecyl-ammonium (DDA-Br ou DDA-CI), et
- un second composant adjuvant hydrophobique, choisi dans le groupe constitué de: lipide monophosphoryle A (MPL-A), dibéhénate de tréhalose (TDB) et un dérivé de sorbitan.

3. Combinaison adjuvant selon la revendication 1 ou 2, dans laquelle le dérivé de sorbitan est le trioléate de sorbitan.

4. Kit de combinaison pour l'immunisation comprenant une combinaison adjuvant selon l'une quelconque des revendications 1 à 3 et une substance antigénique.

5. Kit d'immunisation selon la revendication 4, dans lequel la substance antigénique est dérivée d'un homogénat cellulaire ou des fractions de ce dernier.

6. Kit d'immunisation selon la revendication 4, dans lequel la substance antigénique est un filtrat de culture (CF), comme un filtrat de culture provenant de la culture d'un microorganisme.

7. Kit d'immunisation selon la revendication 4, dans lequel la substance antigénique est dérivée de *Mycobacterium tuberculosis.*

8. Kit d'immunisation selon la revendication 4, dans lequel la substance antigénique est un fragment antigénique, par exemple une espèce moléculaire essentiellement pure.

9. Kit d'immunisation selon la revendication 4, dans lequel l'espèce moléculaire antigénique est d'origine synthétique ou recombinante.

10. Kit d'immunisation selon la revendication 4, dans lequel l'espèce moléculaire antigénique est l'ESAT-6 de *Mycobacterium tuberculosis.*

11. Kit d'immunisation selon la revendication 4, dans lequel l'espèce moléculaire antigénique est une molécule hybride recombinante comprenant l'ESAT-6 de *Mycobacterium tuberculosis.*
